# EUROPEAN PATENT APPLICATION

(11) **EP 4 574 074 A1**
(43) Date of publication of application: **25.06.2025**
(21) Application number: 24220863.5
(22) Date of filing: 18.12.2024
(51) Int. Cl.: A61B 18/14, A61B 5/00

(54) **MAPPING AND ABLATION CATHETER**

(30) Priority: 19.12.2023 US 202318544835
(71) Applicant: Biosense Webster (Israel) Ltd., 2066717 Yokneam (IL)
(72) Inventor: RODRIGUEZ SOTO, Juan, Irvine, 92618 (US); EBRAHIMI, Babak, Irvine, 92618 (US); VAN NIEKERK, Pieter Emmelius, Irvine, 92618 (US); HENRIQUEZ, Jamie, Irvine, 92618 (US)
(74) Representative: Carpmaels & Ransford LLP

(57) **Abstract**

The disclosed technology includes a medical probe configured for mapping and ablation of tissue. The medical probe comprises an inner catheter shaft extending along a longitudinal axis and an ablation electrode disposed at a distal end of the inner catheter shaft. The ablation electrode can be configured to deliver ablative energy to tissue. The medical probe further includes an outer sleeve disposed at least partially around the inner catheter shaft and extending along the longitudinal axis and comprising a plurality of spines disposed at a distal end of the outer sleeve. Each spine of the plurality of spines can comprise a mapping electrode configured to detect electrophysiological signals. The outer sleeve can be configured to move axially along the inner catheter shaft to move the plurality of spines axially with respect to the ablation electrode.

## Description

### Field

The present invention relates generally to medical devices configured for mapping and ablation of tissue.

### BACKGROUND

Cardiac arrhythmias, such as atrial fibrillation (AF), occur when regions of cardiac tissue abnormally conduct electric signals to adjacent tissue. This disrupts the normal cardiac cycle and causes asynchronous rhythm. Certain procedures exist for treating arrhythmia, including surgically disrupting the origin of the signals causing the arrhythmia and disrupting the conducting pathway for such signals. By selectively ablating cardiac tissue by application of energy via a catheter, it is sometimes possible to cease or modify the propagation of unwanted electrical signals from one portion of the heart to another.

To perform ablation of cardiac tissue, a physician generally inserts a sheath catheter through a blood vessel accessed near the groin and navigates the sheath catheter to the patient's heart. Once in the heart, the physician may perform a transeptal puncture to access an opposite side of the heart, if necessary. The physician then generally inserts a mapping catheter and navigates the mapping catheter to an area of interest in the hearth to collect electrophysiological signals from the tissue to generate an electrophysiological map of the tissue. Some example probes include a number of spines with electrodes positioned thereon. The electrodes are generally attached to the spines and the spines are configured to extend outwardly when deployed from a sheath. The electrodes are then brought into contact with tissue for mapping of electrophysiological signals propagating through the tissue.

Once a region of the heart is identified as propagating aberrant electrical signals, the physician generally must remove the mapping catheter and then insert an ablation catheter to perform the ablation. As before, the physician will navigate the ablation catheter to the area of interest. Once the ablation catheter is properly positioned at the area of interest, ablative energy can be delivered to the tissue to disrupt the propagation of aberrant electrical signals. As will be appreciated, however, inserting more than one catheter elongates the surgery time which can be difficult for the physician and patient alike.

To shorten the surgery time, some physicians will insert more than one catheter at a time, which may require multiple access points and transeptal punctures. Long surgery times and multiple access points or transeptal punctures can elongate the recovery time for the patient. What is needed, therefore, is a device and method for reducing the complexity and time required for performing mapping and ablation of cardiac tissue. These and other problems can be addressed by the technology disclosed herein.

### SUMMARY

There is provided, in accordance with the disclosed technology, a medical probe configured for mapping and ablation of tissue. The medical probe comprises an inner catheter shaft extending along a longitudinal axis and an ablation electrode disposed at a distal end of the inner catheter shaft. The ablation electrode can be configured to deliver ablative energy to tissue. The medical probe further includes an outer sleeve disposed at least partially around the inner catheter shaft and extending along the longitudinal axis and comprising a plurality of spines disposed at a distal end of the outer sleeve. Each spine of the plurality of spines can comprise a mapping electrode configured to detect electrophysiological signals. The outer sleeve can be configured to move axially along the inner catheter shaft to move the plurality of spines axially with respect to the ablation electrode.

The disclosed technology further includes a method of performing mapping and ablation of tissue. The method can comprise inserting a medical probe into a lumen of a body. The medical probe can comprise an inner catheter shaft extending along a longitudinal axis and an ablation electrode disposed at a distal end of the inner catheter shaft. The ablation electrode can be configured to deliver ablative energy to tissue. The medical probe can further include an outer sleeve disposed at least partially around the inner catheter shaft and extending along the longitudinal axis, and a plurality of spines disposed at a distal end of the outer sleeve. Each spine of the plurality of spines can comprise a mapping electrode configured to detect electrophysiological signals.

The method can further include sliding the outer sleeve along the inner catheter shaft to cause the plurality of spines to extend beyond a distal end of the ablation electrode, and receiving one or more electrophysiological signals from at least one mapping electrode disposed on the plurality of spines.

### BRIEF DESCRIPTION OF THE DRAWINGS

The present disclosure will be more fully understood from the following detailed description of embodiments thereof, taken together with the drawings, in which:
FIG. 1 is a schematic pictorial illustration of a medical system including a medical probe, in accordance with the disclosed technology;
FIG. 2A is a schematic pictorial illustration of the medical probe with an end effector and handle in a first configuration, in accordance with the disclosed technology;
FIG. 2B is a schematic pictorial illustration of the medical probe with an end effector and handle in a second configuration, in accordance with the disclosed technology;
FIG. 3A is a side view of the end effector, in accordance with the disclosed technology;
FIG. 3B is a side view of the end effector with a mapping assembly pushed distally, in accordance with the disclosed technology;
FIG. 3C is a side view of the end effector with a mapping assembly pulled proximally, in accordance with the disclosed technology;
FIG. 4 is a detail view of the end effector, in accordance with the disclosed technology;
FIG. 5A is a top perspective view of a mapping assembly, in accordance with the disclosed technology;
FIG. 5B is a top view of a mapping assembly, in accordance with the disclosed technology;
FIG. 5C is a side view of a mapping assembly, in accordance with the disclosed technology;
FIG. 6 is a side view of a mapping assembly and outer sleeve, in accordance with the disclosed technology;
FIG. 7A is a perspective view of an ablation assembly and inner catheter shaft, in accordance with the disclosed technology;
FIG. 7B is a side view of an ablation assembly and inner catheter shaft, in accordance with the disclosed technology; and
FIG. 8 is a flow chart of a method of performing mapping and ablation of tissue, in accordance with the disclosed technology.

### DETAILED DESCRIPTION

The disclosed technology relates to a medical probe configured for performing mapping of electrophysiological signals and ablation of tissue. The disclosed medical probe can simplify the processes required for mapping and ablating tissue by eliminating the need for using more than one catheter. To illustrate, the disclosed technology includes an end effector having a mapping assembly disposed at least partially around an ablation assembly. The mapping assembly can be moved distally beyond a distal end of the ablation assembly such that the physician can contact tissue and obtain electrophysiological signals from the tissue. The mapping assembly can also be moved proximally such that the ablation assembly is distal to a distal end of the mapping assembly so that the ablation assembly can deliver ablative energy to tissue. Stated otherwise, a physician can slide the mapping assembly distally to perform a mapping procedure and then slide the mapping assembly proximally to expose an ablation catheter and perform an ablation procedure. The mapping and ablation procedures can be done with the single medical probe, thereby simplifying the surgery workflow and reducing the overall surgery time. Reducing the surgery time can also help to reduce the recovery time of the patient.

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%.

In addition, as used herein, the terms "patient," "host," "user," and "subject" refer to any human or animal subject and are not intended to limit the systems or methods to human use, although use of the subject invention in a human patient represents a preferred embodiment. As well, the term "proximal" indicates a location closer to the operator or physician whereas "distal" indicates a location further away from the operator or physician.

As discussed herein, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "physician" can include a doctor, surgeon, technician, scientist, operator, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as irreversible electroporation (IRE), referred throughout this disclosure interchangeably as pulsed electric field (PEF) and pulsed field ablation (PFA). Ablating or ablation as it relates to the devices and corresponding systems of this disclosure is used throughout this disclosure in reference to non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "bipolar" and "unipolar" when used to refer to ablation schemes describe ablation schemes which differ with respect to electrical current path and electric field distribution. "Bipolar" refers to an ablation scheme utilizing a current path between two electrodes that are both positioned at a treatment site; current density and electric flux density is typically approximately equal at each of the two electrodes. "Unipolar" refers to ablation scheme utilizing a current path between two electrodes where one electrode having a high current density and high electric flux density is positioned at a treatment site, and a second electrode having comparatively lower current density and lower electric flux density is positioned remotely from the treatment site.

As discussed herein, the terms "biphasic pulse" and "monophasic pulse" refer to respective electrical signals. "Biphasic pulse" refers to an electrical signal having a positive-voltage phase pulse (referred to herein as "positive phase") and a negative-voltage phase pulse (referred to herein as "negative phase"). "Monophasic pulse" refers to an electrical signal having only a positive or only a negative phase. Preferably, a system providing the biphasic pulse is configured to prevent application of a direct current voltage (DC) to a patient. For instance, the average voltage of the biphasic pulse can be zero volts with respect to ground or other common reference voltage. Additionally, or alternatively, the system can include a capacitor or other protective component. Where voltage amplitude of the biphasic and/or monophasic pulse is described herein, it is understood that the expressed voltage amplitude is an absolute value of the approximate peak amplitude of each of the positive-voltage phase and/or the negative-voltage phase. Each phase of the biphasic and monophasic pulse preferably has a square shape having an essentially constant voltage amplitude during a majority of the phase duration. Phases of the biphasic pulse are separated in time by an interphase delay. The interphase delay duration is preferably less than or approximately equal to the duration of a phase of the biphasic pulse. The interphase delay duration is more preferably about 25% of the duration of the phase of the biphasic pulse.

As discussed herein, the terms "tubular" and "tube" are to be construed broadly and are not limited to a structure that is a right cylinder or strictly circumferential in cross-section or of a uniform cross-section throughout its length. For example, the tubular structures are generally illustrated as a substantially right cylindrical structure. However, the tubular structures may have a tapered or curved outer surface without departing from the scope of the present disclosure.

The present disclosure is related to systems, method or uses and devices that can be used for IRE ablation of cardiac tissue to treat cardiac arrhythmias. Ablative energies are typically provided to cardiac tissue by a tip portion of a catheter which can deliver ablative energy alongside the tissue to be ablated. Some example catheters include three-dimensional structures at the tip portion and are configured to administer ablative energy from various electrodes positioned on the three-dimensional structures. Ablative procedures incorporating such example catheters can be visualized using fluoroscopy.

Ablation of cardiac tissue using application of a thermal technique, such as radio frequency (RF) energy and cryoablation, to correct a malfunctioning heart is a well-known procedure. Typically, to successfully ablate using a thermal technique, cardiac electropotentials need to be measured at various locations of the myocardium. In addition, temperature measurements during ablation provide data enabling the efficacy of the ablation. Typically, for an ablation procedure using a thermal technique, the electropotentials and the temperatures are measured before, during, and after the actual ablation. RF approaches can have risks that can lead to tissue charring, burning, steam pop, phrenic nerve palsy, pulmonary vein stenosis, and esophageal fistula. Cryoablation is an alternative approach to RF ablation that can reduce some thermal risks associated with RF ablation. However, maneuvering cryoablation devices and selectively applying cryoablation is generally more challenging compared to RF ablation; therefore, cryoablation is not viable in certain anatomical geometries which may be reached by electrical ablation devices.

The present disclosure can include electrodes configured for RF ablation, cryoablation, and/or irreversible electroporation (IRE). IRE can be referred to throughout this disclosure interchangeably as pulsed electric field (PEF) ablation and pulsed field ablation (PFA). IRE as discussed in this disclosure is a non-thermal cell death technology that can be used for ablation of atrial arrhythmias. To ablate using IRE/PEF, biphasic voltage pulses are applied to disrupt cellular structures of myocardium. The biphasic pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area. IRE, therefore, has the capability to spare adjacent heat sensitive structures or tissues which would be of benefit in the reduction of possible complications known with ablation or isolation modalities. Additionally, or alternatively, monophasic pulses can be utilized.

Electroporation can be induced by applying a pulsed electric field across biological cells to cause reversable (temporary) or irreversible (permanent) creation of pores in the cell membrane. The cells have a transmembrane electrostatic potential that is increased above a resting potential upon application of the pulsed electric field. While the transmembrane electrostatic potential remains below a threshold potential, the electroporation is reversable, meaning the pores can close when the applied pulse electric field is removed, and the cells can self-repair and survive. If the transmembrane electrostatic potential increases beyond the threshold potential, the electroporation is irreversible, and the cells become permanently permeable. As a result, the cells die due to a loss of homeostasis and typically die by apoptosis. Generally, cells of differing types have differing threshold potential. For instance, heart cells have a threshold potential of approximately 500 V/cm, whereas for bone it is 3000 V/cm. These differences in threshold potential allow IRE to selectively target tissue based on threshold potential.

The solution of this disclosure includes systems and methods for applying electrical signals from catheter electrodes positioned in the vicinity of myocardial tissue to generate an ablative energy to ablate the myocardial tissue. In some examples, the systems and methods can be effective to ablate targeted tissue by inducing irreversible electroporation. In some examples, the systems and methods can be effective to induce reversible electroporation as part of a diagnostic procedure. Reversible electroporation occurs when the electricity applied with the electrodes is below the electric field threshold of the target tissue allowing cells to repair. Reversible electroporation does not kill the cells but allows a physician to see the effect of reversible electroporation on electrical activation signals in the vicinity of the target location. Example systems and methods for reversible electroporation is disclosed in U.S. Patent Publication 2021/0162210, the entirety of which is incorporated herein by reference.

The pulsed electric field, and its effectiveness to induce reversible and/or irreversible electroporation, can be affected by physical parameters of the system and biphasic pulse parameters of the electrical signal. Physical parameters can include electrode contact area, electrode spacing, electrode geometry, etc. Examples presented herein generally include physical parameters adapted to effectively induce reversible and/or irreversible electroporation. Biphasic pulse parameters of the electrical signal can include voltage amplitude, pulse duration, pulse interphase delay, inter-pulse delay, total application time, delivered energy, etc. In some examples, parameters of the electrical signal can be adjusted to induce both reversible and irreversible electroporation given the same physical parameters. Examples of various systems and methods of ablation including IRE are presented in U.S. Patent Publications 2021/0169550A1, 2021/0169567A1, 2021/0169568A1, 2021/0161592A1, 2021/0196372A1, 2021/0177503A1, and 2021/0186604A1, the entireties of each of which are incorporated herein by reference.

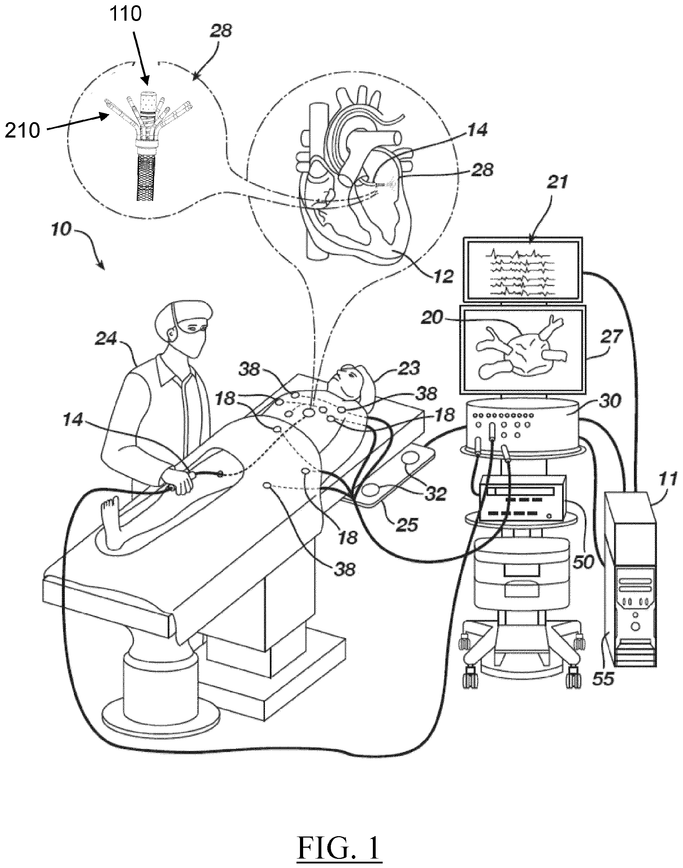

Reference is made to FIG. 1 showing an example catheter-based electrophysiology mapping and ablation system 10. System 10 includes multiple catheters, which are percutaneously inserted by physician 24 through the patient's 23 vascular system into a chamber or vascular structure of a heart 12. Typically, a delivery sheath catheter is inserted into the left or right atrium near a desired location in heart 12. Thereafter, a catheter having an end effector at a distal tip 28 is inserted into the delivery sheath catheter so as to arrive at the desired location. An example catheter 14 that is configured for sensing and ablating is illustrated and described further herein. Physician 24 brings a distal tip 28 (sometimes referred to herein as end effector 28) of catheter 14 into contact with the heart wall for sensing a target site in heart 12 using a mapping assembly (as will be described further here). For ablation, the physician 24 can move the mapping assembly proximally to expose an ablation assembly (as will be described further here) for ablating the tissue.

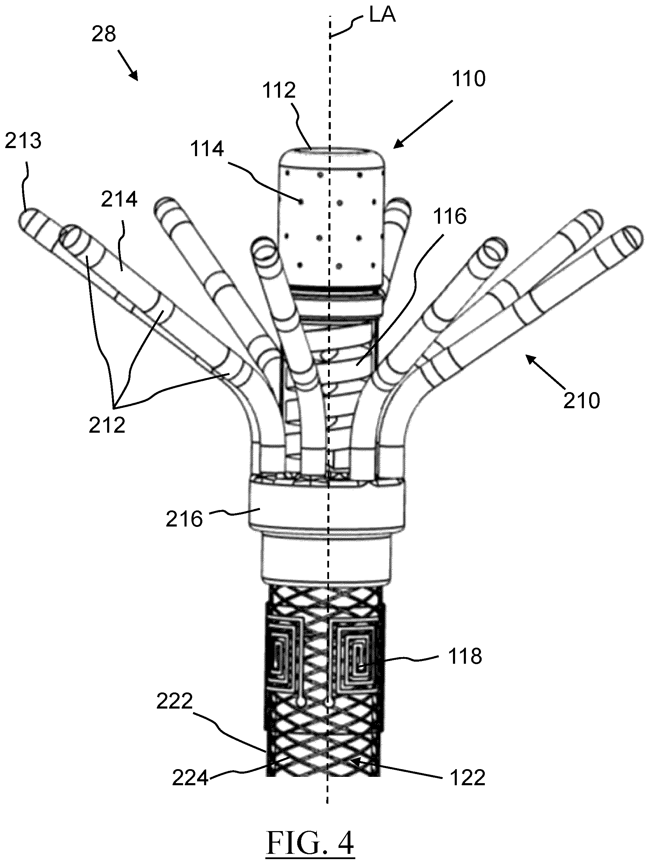

Catheter 14 is an exemplary catheter that includes one and preferably multiple electrodes 212 optionally distributed over a plurality of spines 214 at distal tip 28 (as shown in FIG. 4) and configured to sense the IEGM signals and/or to ablate tissue. Catheter 14 may additionally include a position sensor 118 embedded in or near distal tip 28 for tracking position and orientation of distal tip 28. Optionally and preferably, position sensor 118 is a magnetic based position sensor including three magnetic coils for sensing three-dimensional (3D) position and orientation.

Magnetic based position sensor 118 may be operated together with a location pad 25 including a plurality of magnetic coils 32 configured to generate magnetic fields in a predefined working volume. Real time position of distal tip 28 of catheter 14 may be tracked based on magnetic fields generated with location pad 25 and sensed by magnetic based position sensor 118. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091, the entireties of each of which are incorporated herein by reference.

System 10 includes one or more electrode patches 38 positioned for skin contact on patient 23 to establish location reference for location pad 25 as well as impedance-based tracking of electrodes 26. For impedance-based tracking, electrical current is directed toward electrodes 26 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182, the entireties of each of which are incorporated herein by reference.

A recorder 11 displays electrograms 21 captured with body surface ECG electrodes 18 and intracardiac electrograms (IEGM) captured with electrodes 26 of catheter 14. Recorder 11 may include pacing capability for pacing the heart rhythm and/or may be electrically connected to a standalone pacer.

System 10 may include an ablation energy generator 50 that is adapted to conduct ablative energy to one or more of electrodes at a distal tip of a catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between catheters, electrophysiological equipment, power supply and a workstation 55 for controlling operation of system 10. Electrophysiological equipment of system 10 may include for example, catheter 14, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (4) displaying on display device 27 sites of interest such as places where ablation energy has been applied. One commercial product embodying elements of the system 10 is available as the CARTO^{™} 3 System, available from Biosense Webster, Inc., 31 Technology Drive, Suite 200, Irvine, CA 92618, USA.

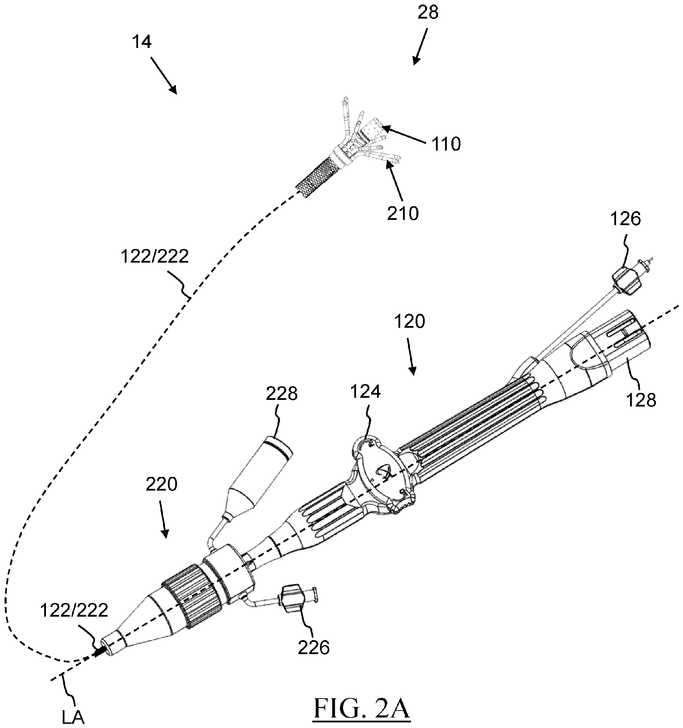

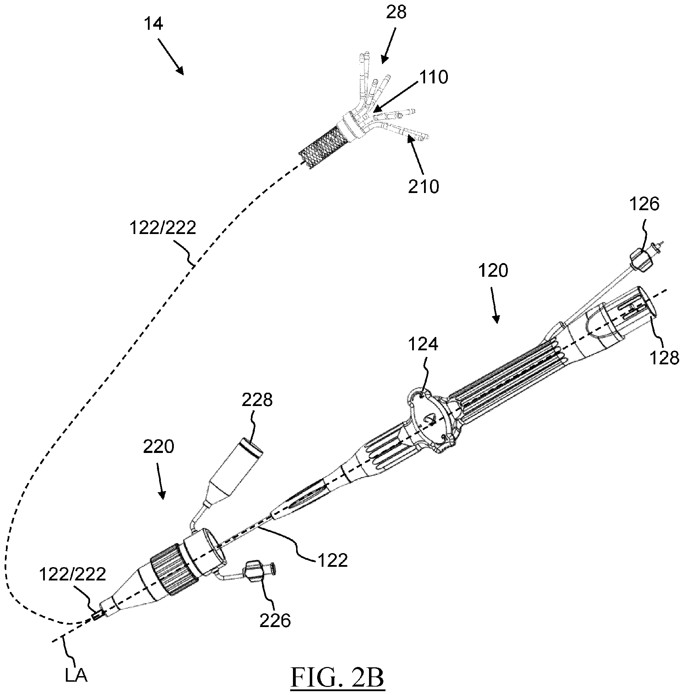

FIGs. 2A and 2B show the catheter 14 in greater detail. The catheter 14 (sometimes referred to herein as a medical probe) can be configured to be inserted into a heart 12 of a patient 23 for mapping and ablation of tissue in the heart 12. As shown, the catheter 14 can include an end effector 28, a first handle 120, and a second handle 220. The end effector 28 can be attached to the first handle 120 by an inner catheter shaft 122 and the second handle 220 by an outer sleeve 222. The sleeve 222 can be disposed around the inner catheter shaft 122 such that the sleeve 222 can slide along the outside of the inner catheter shaft 122.

The end effector 28 can further include an ablation assembly 110 and a mapping assembly 210. The ablation assembly 110 is attached to a distal end of the inner catheter shaft 122 while the mapping assembly 210 is attached to a distal end of the sleeve 222. In this way, as a physician 24 slides the first handle 120 and the second handle 220 toward each other (as shown in FIG. 2A), the ablation assembly 110 can extend beyond a distal end of the mapping assembly 210 such that the end effector 28 is configured for delivering ablative energy to tissue. Alternatively, when the physician moves the first handle 120 and the second handle 220 away from each other (as shown in FIG. 2B), the mapping assembly 210 will slide beyond a distal end of the ablation assembly 110 such that the mapping assembly 210 is configured to contact tissue and collect electrophysiological signals from the tissue. In other words, the physician 24 can slide the second handle 220 distally to move the mapping assembly 210 into contact with tissue to perform mapping of electrophysiological signals and the physician 24 can slide the second handle 220 proximally to expose the ablation assembly 110 to perform an ablation procedure.

The first handle 120 can include an actuator 124 that can be configured to cause the end effector 28 to deflect radially outward from a longitudinal axis LA. The actuator 124, for example, can be attached to a pull wire that is attached to a distal end of the inner catheter shaft 122 or to the ablation assembly 110. As the physician 24 actuates the actuator 124, the pull wire will be pulled to cause the end effector 28 to deflect outward from the longitudinal axis LA. As will be appreciated, as the inner catheter shaft 122 is deflected outwardly, the inner catheter shaft 122 will also cause the sleeve 222 (which is disposed around the inner catheter shaft 122) to deflect outwardly. In this way, both the ablation assembly 110 and the mapping assembly 210 will be caused to deflect outwardly from the longitudinal axis LA when the actuator 124 is actuated.

The first handle 120 can further comprise a first irrigation coupler 126 and a first electrical coupler 128. The first irrigation coupler 126 can be configured to connect to an irrigation supply and deliver irrigation through the first handle 120 and to the ablation assembly 110. As shown in FIG. 4, the ablation assembly 110 can include irrigation holes 114 configured to deliver irrigation fluid to the tissue near the end effector 28. The first electrical coupler 128 can be configured to connect to a corresponding electrical coupler to connect the catheter 14 to the PIU 30 so that ablative energy supplied by the ablation energy generator 50 can be delivered to the ablation assembly 110.

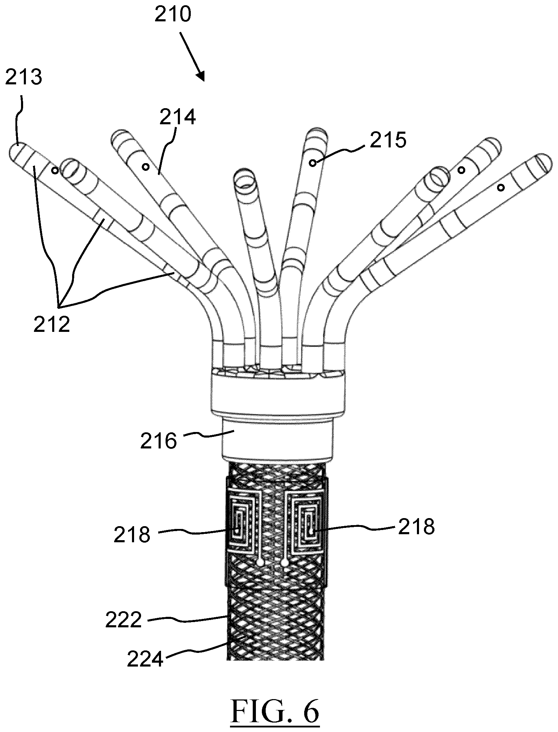

The second handle 220 can comprise a second irrigation coupler 226 and a second electrical coupler 228. The second irrigation coupler 226 can be configured to connect to an irrigation supply and deliver irrigation through the second handle 220 and to the mapping assembly 210. As shown in FIG. 6, the mapping assembly 210 can include irrigation holes 215 configured to deliver irrigation fluid to the tissue near the mapping assembly 210. The second electrical coupler 228 can be configured to connect to a corresponding electrical coupler to connect the catheter 14 to the PIU 30 so that electrical signals detected by the mapping assembly 210 can be analyzed and output for display.

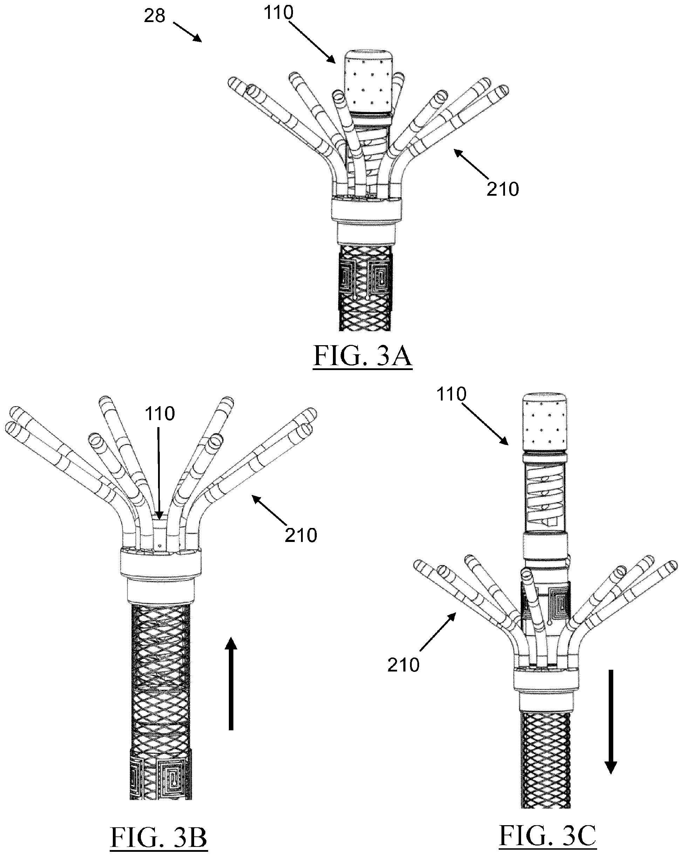

FIGs. 3A, 3B, and 3C show additional view of the end effector 28. As shown, the mapping assembly 210 can be disposed generally around the ablation assembly 110 and be configured to slide distally and proximally over the ablation assembly 110 along the longitudinal axis. As illustrated in FIG. 3B, when the mapping assembly 210 is moved distally, much of the mapping assembly 210 is moved beyond a distal end of the ablation assembly 110. In this way, the mapping assembly 210 can be configured to contact tissue and detect electrophysiological signals propagating through the tissue to perform a mapping procedure without being impeded by the ablation assembly 110. In contrast, when the mapping assembly 210 is pulled proximally, the ablation assembly 110 will extend beyond a distal end of the mapping assembly 210 such that that the ablation assembly 110 is configured to deliver ablative energy to tissue without being impeded by the mapping assembly 210. In this way, the end effector 28 can be configured for performing both a mapping procedure and an ablation procedure with only the single catheter 14.

As shown in FIG. 4, the ablation assembly 110 can be disposed at a distal end of the inner catheter shaft 122 and comprise an ablation electrode 112. In this example, the ablation electrode 112 is a single electrode disposed at the distal end of the inner catheter shaft 122, but it will be appreciated that more than one electrode can be disposed at the distal end of the inner catheter shaft 122 depending on the particular configuration. The ablation assembly 110 can be configured to perform RF ablation, IRE ablation, and/or cryoablation. If the ablation assembly 110 is configured to perform IRE ablation, the ablation electrode 112 (or electrodes, depending on the configuration) can be configured to deliver unipolar or bipolar ablation schemes. Furthermore, the ablation electrode 112 can be configured to deliver monophasic or biphasic ablation pulses.

As briefly described above, the ablation assembly 110 can further include irrigation holes 114 configured to deliver irrigation fluid to the ablation assembly 110, the mapping assembly 210, and/or the tissue. The irrigation holes 114 can be disposed circumferentially around the ablation electrode 112 and be configured to direct irrigation fluid outwardly from the ablation electrode 112.

The ablation assembly 110 can further include a force sensor 116 that is configured to detect a force applied to the ablation assembly 110. The force sensor 116 can be configured to detect a force applied to the distal end of the ablation assembly 110 such as when the ablation assembly 110 is in contact with tissue. In this way, the force sensor 116 can help a physician 24 to know when the ablation assembly 110 is in sufficient contact with the tissue for delivering ablative energy to the tissue. Examples of contact force sensor assemblies are disclosed in U.S. Patent Nos. 8,357,152 and 10,688,278 and U.S. Patent Pub. No. 2021/0187254A1, each of which are incorporated herein by reference and attached in the appendix included herewith.

As shown in FIG. 4, the mapping assembly 210 can be disposed at a distal end of the sleeve 222. The sleeve 222 comprises a braided structure 224 such that the sleeve 222 is prevented from buckling and/or becoming pinched when manipulated. The braided structure 224 can further comprise electrical wires that can be connected to the mapping electrodes 212 and/or the ablation electrode 112. As will be appreciated, by incorporating electrical wires into the braided structure 224, the overall profile of the catheter 14 can be reduced. The mapping assembly 210 can further include one or more position sensors 118 that are disposed near the mapping assembly 210. The position sensors 118 can be configured to detect a position and orientation of the end effector 28 and that information can be output to the display device 27 for display to the physician 24. The position sensors 118 can be any suitable type of position sensors. For example, and not limitation, the position sensors 118 can be one or more electromagnetic coils configured to output a signal when subjected to a generated electromagnetic field. In some examples, the position sensors 118 can be three position sensors that are disposed approximately 120 degrees apart from each other around the circumference of the sleeve 222. Although the position sensors 118 are shown as being disposed on the sleeve 222, it will be appreciated that the position sensors 118 can, alternatively or in addition, be disposed on the inner catheter shaft 122.

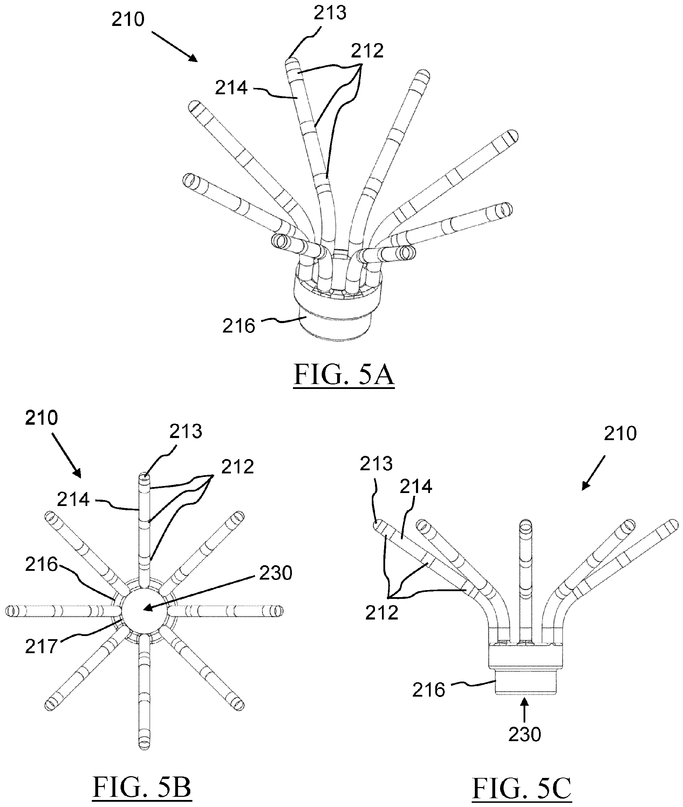

As further shown in FIGs. 5A, 5B, and 5C, the mapping assembly 210 can include a spine hub 216 and a plurality of spines 214 extending radially outward from the spine hub 216. The spines 214 can each include one or more mapping electrodes 212 that are configured to detect electrophysiological signals propagating through tissue. The spines 214 can be configured to extend generally in a distal direction such that the spines 214 and electrodes 212 contact tissue when the mapping assembly 210 is brought into contact with tissue.

It will be appreciated that the mapping assembly 210 can include various different numbers of spines 214. For example, the mapping assembly 210 can include three spines, four spines, five spines, six spines, seven spines, eight spines, nine spines, ten spines, fifteen spines, twenty spines, or any other suitable number of spines for the particular configuration. As shown in FIGs. 5A-5C the mapping assembly 210 can include eight spines as a specific example.

The spine hub 216 can comprise a lumen 230 that extends through the spine hub 216 along the longitudinal axis LA. The lumen 230 can be sized to fit around the ablation assembly 110 such that the ablation assembly 110 can slide through the spine hub 216.

In some examples, the spine hub 216 can include one or more connectors 217 that can be configured to electrically connect one or more mapping electrodes 212 to the ablation electrode 112. When the ablation electrode 112 is in electrical communication with the connector 217, the electrical energy delivered to the ablation electrode 112 can be distributed to the one or more mapping electrodes 212 to deliver ablative energy through the one or more electrodes 212. For example, when the ablation electrode 112 is in electrical communication with connector 217, all of the mapping electrodes 212, only one mapping electrodes 212, each distally-positioned mapping electrode 212, every other mapping electrode 212, or any other configuration of mapping electrodes 212 can be configured to deliver ablative energy. As will be appreciated, by delivering ablative energy through a plurality of the mapping electrodes 212, a greater area of tissue can be ablated.

FIG. 6 illustrates an alternate example of a mapping assembly 210 having irrigation holes 215 disposed along the spines 214. The spines 214, for example, can include a tube or sleeve disposed along or around the spines 214 to deliver irrigation fluid to the irrigation holes 215. Alternatively, the spines 214 themselves can be made from a hollow tube stock and be configured to deliver the irrigation fluid to the irrigation holes 215. The irrigation holes 215 can be configured to deliver irrigation fluid from an irrigation supply as previously described. In some examples, the irrigation holes 215 can be oriented such that the irrigation fluid is delivered to the tissue near the distal ends of the spines 214. Furthermore, although only one irrigation hole 215 is shown on each spine 214, it will be appreciated that more than one irrigation hole 215 can be disposed on each spine 214 and be configured to direct irrigation fluid to desired locations (e.g., toward the tissue, toward the ablation assembly 110, toward the spine hub 216).

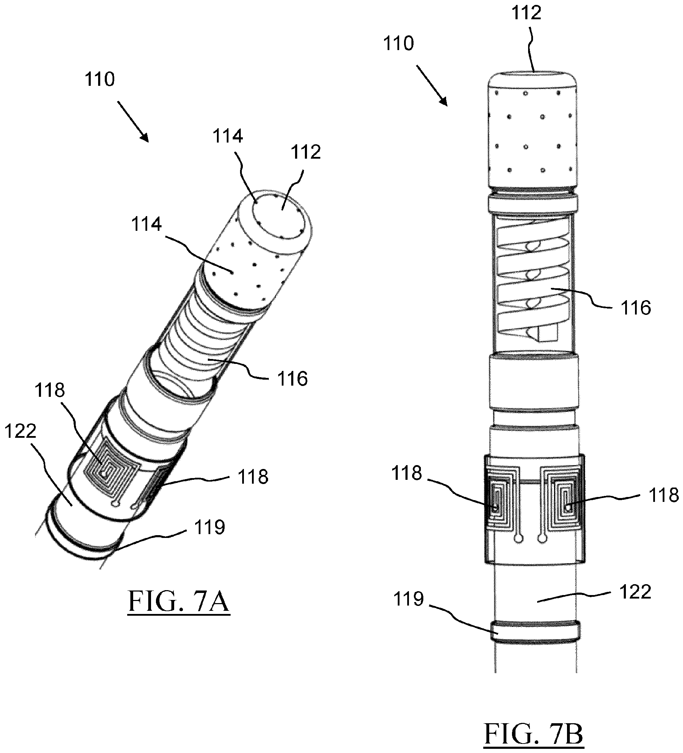

As shown in FIGs. 7A and 7B, the ablation assembly 110 can be disposed at a distal end of the inner catheter shaft 122 and can include an ablation electrode 112, irrigation holes 114 disposed on the ablation electrode 112 (radially around the circumference as well as on the distal tip), a force sensor 116, position sensors 118, and a marker band 119. As previously described, the force sensor 116 can be configured to detect a force applied to the ablation electrode 112 such that feedback can be provided to the physician 24 to indicate when the ablation electrode 112 is in sufficient contact with tissue. The position sensors 118 can be the same or similar as the position sensors 218 previously described. For example, the position sensors 118 can be electromagnetic coils disposed approximately 120 degrees apart from each other around the circumference of the inner catheter shaft 122. The marker band 119 can be a radiopaque marker configured for use with fluoroscopy to determine a position of the end effector 28.

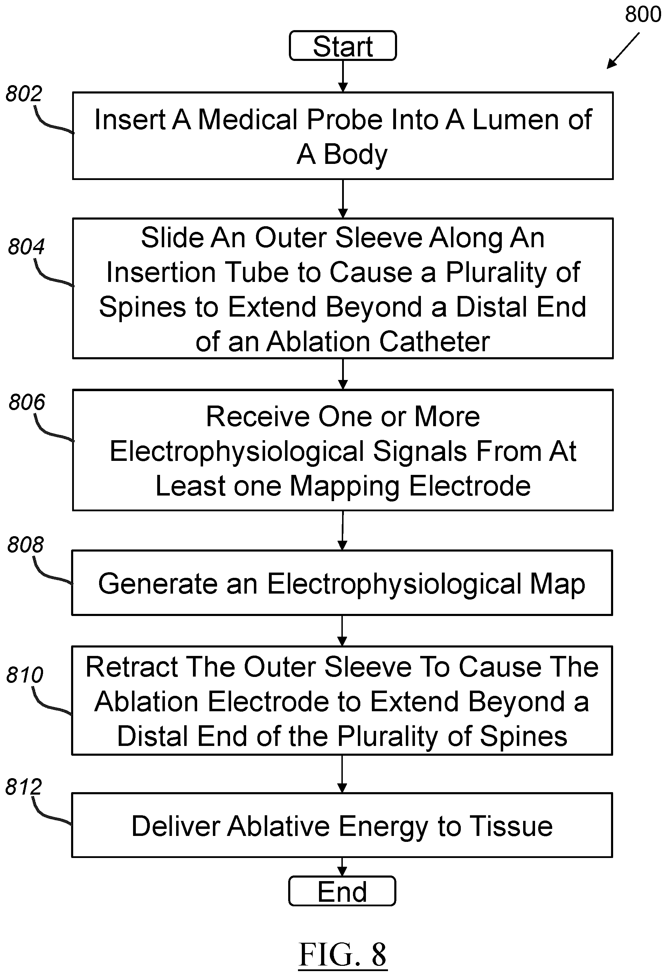

FIG. 8 is a flow chart of a method 800 of performing mapping and ablation of tissue, in accordance with the disclosed technology. The method 800 can include inserting 802 a medical probe into a lumen of a body, sliding 804 an outer sleeve along an inner catheter shaft to cause a plurality of spines to extend beyond a distal end of an ablation catheter, and receiving 806 one or more electrophysiological signals from at least one mapping electrode. As just described, the method 800 can be used for completing a mapping procedure to determine the location and direction of electrophysiological signals propagated through tissue. The collection of the electrophysiological signals can be used to identify the location and direction of aberrant signals propagated through tissue.

The method 800 can further include retracting 810 the outer sleeve to cause the ablation electrode to extend beyond a distal end of the plurality of spines and delivering 812 ablative energy to tissue. In other words, the method 800 can include completing an ablation procedure. The ablation procedure can include ablating tissue at identified locations to stop the propagation of the aberrant signals through the tissue.

As will be appreciated, the method 800 just described is not intended to be limited to the particular steps described or to be limited to the particular order of steps described. That is, the method 800 can include other intervening steps that are not explicitly described herein and/or can be executed in various orders. Accordingly, although the method 800 is described as having particular steps and is presented in a particular an order, the method 800 is not so limited.

The disclosed technology described herein can be further understood according to the following clauses:
Clause 1: A medical probe comprising: an inner catheter shaft extending along a longitudinal axis; an ablation electrode disposed at a distal end of the inner catheter shaft, the ablation electrode configured to deliver ablative energy to tissue; an outer sleeve disposed at least partially around the inner catheter shaft and extending along the longitudinal axis; and a plurality of spines disposed at a distal end of the outer sleeve, each spine of the plurality of spines comprising a mapping electrode configured to detect electrophysiological signals, the outer sleeve configured to move axially along the inner catheter shaft to move the plurality of spines axially with respect to the ablation electrode.
Clause 2: The medical probe of clause 1, the outer sleeve configured to move the plurality of spines beyond a distal end of the ablation electrode.
Clause 3: The medical probe of clause 2, the plurality of spines configured to extend radially outward from the longitudinal axis.
Clause 4: The medical probe of clause 3, the plurality of spines configured to be positioned radially around the ablation electrode.
Clause 5: The medical probe of clause 1, further comprising a first handle disposed at a proximal end of the inner catheter shaft and a second handle disposed at a proximal end of the outer sleeve.
Clause 6: The medical probe of clause 5, the second handle configured to be moved axially along the longitudinal axis with respect to the first handle, thereby causing the outer sleeve to move axially along the inner catheter shaft.
Clause 7: The medical probe of clause 5, further comprising a pull wire configured to cause the inner catheter shaft to deflect radially outward from the longitudinal axis.
Clause 8: The medical probe of clause 7, the first handle comprising an actuator configured to pull the pull wire to cause the inner catheter shaft to deflect radially outward from the longitudinal axis.
Clause 9: The medical probe of clause 7, the outer sleeve configured to deflect radially outward when the inner catheter shaft is caused to deflect radially outward from the longitudinal axis.
Clause 10: The medical probe of clause 5, the first handle comprising an irrigation coupler configured to receive irrigation fluid from an irrigation supply and the ablation electrode comprising a plurality of apertures each configured to permit the irrigation fluid to pass therethrough.
Clause 11: The medical probe of clause 5, the second handle comprising an irrigation coupler configured to receive irrigation fluid from an irrigation supply, the outer sleeve configured to deliver irrigation fluid to the plurality of spines.
Clause 12: The medical probe of clause 11, each spine of the plurality of spines comprising an aperture configured to permit the irrigation fluid to pass therethrough.
Clause 13: The medical probe of clause 1, further comprising an electromagnetic sensor disposed near the distal end of the inner catheter shaft, the electromagnetic sensor configured to generate a current when subjected to an electromagnetic field.
Clause 14: The medical probe of clause 1, further comprising a force sensor disposed proximal to the ablation electrode, the force sensor configured to detect a force applied to the ablation electrode.
Clause 15: The medical probe of clause 1, wherein the plurality of spines comprises at least five spines, each spine of the at least five spines comprising a plurality of mapping electrodes.
Clause 16: A method comprising: inserting a medical probe into a lumen of a body, the medical probe comprising: an inner catheter shaft extending along a longitudinal axis; an ablation electrode disposed at a distal end of the inner catheter shaft, the ablation electrode configured to deliver ablative energy to tissue; an outer sleeve disposed at least partially around the inner catheter shaft and extending along the longitudinal axis; and a plurality of spines disposed at a distal end of the outer sleeve, each spine of the plurality of spines comprising a mapping electrode configured to detect electrophysiological signals; sliding the outer sleeve along the inner catheter shaft to cause the plurality of spines to extend beyond a distal end of the ablation electrode; and receiving one or more electrophysiological signals from at least one mapping electrode disposed on the plurality of spines.
Clause 17: The method of clause 16 further comprising generating an electrophysiological map based at least in part on the one or more electrophysiological signals.
Clause 18: The method of clause 16 further comprising actuating a pull wire to cause the inner catheter shaft, ablation electrode, outer sleeve, and the plurality of spines to deflect radially outward away from the longitudinal axis.
Clause 19: The method of clause 16 further comprising retracting the outer sleeve along the inner catheter shaft to cause the ablation electrode to extend beyond a distal end of the plurality of spines.
Clause 20: The method of clause 19 further comprising delivering ablative energy to tissue via the ablation electrode.

The embodiments described above are cited by way of example, and the present invention is not limited by what has been particularly shown and described hereinabove. Rather, the scope of the invention includes both combinations and sub combinations of the various features described hereinabove, as well as variations and modifications thereof which would occur to persons skilled in the art upon reading the foregoing description and which are not disclosed in the prior art.

## Claims

1. A medical probe comprising:
an inner catheter shaft extending along a longitudinal axis;
an ablation electrode disposed at a distal end of the inner catheter shaft, the ablation electrode configured to deliver ablative energy to tissue;
an outer sleeve disposed at least partially around the inner catheter shaft and extending along the longitudinal axis; and
a plurality of spines disposed at a distal end of the outer sleeve, each spine of the plurality of spines comprising a mapping electrode configured to detect electrophysiological signals, the outer sleeve configured to move axially along the inner catheter shaft to move the plurality of spines axially with respect to the ablation electrode.

2. The medical probe of claim 1, the outer sleeve configured to move the plurality of spines beyond a distal end of the ablation electrode.

3. The medical probe of claim 2, the plurality of spines configured to extend radially outward from the longitudinal axis.

4. The medical probe of claim 3, the plurality of spines configured to be positioned radially around the ablation electrode.

5. The medical probe of claim 1, further comprising a first handle disposed at a proximal end of the inner catheter shaft and a second handle disposed at a proximal end of the outer sleeve.

6. The medical probe of claim 5, the second handle configured to be moved axially along the longitudinal axis with respect to the first handle, thereby causing the outer sleeve to move axially along the inner catheter shaft.

7. The medical probe of claim 5, further comprising a pull wire configured to cause the inner catheter shaft to deflect radially outward from the longitudinal axis.

8. The medical probe of claim 7, the first handle comprising an actuator configured to pull the pull wire to cause the inner catheter shaft to deflect radially outward from the longitudinal axis.

9. The medical probe of claim 7, the outer sleeve configured to deflect radially outward when the inner catheter shaft is caused to deflect radially outward from the longitudinal axis.

10. The medical probe of claim 5, the first handle comprising an irrigation coupler configured to receive irrigation fluid from an irrigation supply and the ablation electrode comprising a plurality of apertures each configured to permit the irrigation fluid to pass therethrough.

11. The medical probe of claim 5, the second handle comprising an irrigation coupler configured to receive irrigation fluid from an irrigation supply, the outer sleeve configured to deliver irrigation fluid to the plurality of spines.

12. The medical probe of claim 11, each spine of the plurality of spines comprising an aperture configured to permit the irrigation fluid to pass therethrough.

13. The medical probe of claim 1, further comprising an electromagnetic sensor disposed near the distal end of the inner catheter shaft, the electromagnetic sensor configured to generate a current when subjected to an electromagnetic field, or a force sensor disposed proximal to the ablation electrode, the force sensor configured to detect a force applied to the ablation electrode.

14. The medical probe of claim 1, wherein the plurality of spines comprises at least five spines, each spine of the at least five spines comprising a plurality of mapping electrodes.
